# EUROPEAN PATENT SPECIFICATION

(11) **EP 4 563 990 B1**
(45) Date of publication and mention of the grant of the patent: **17.06.2026**
(21) Application number: 23212527.8
(22) Date of filing: 28.11.2023
(51) Int. Cl.: G01N 23/2251, H01J 37/28

(54) **ELECTRON TOMOGRAPHY STAGE CONTROL**
STEUERUNG EINES ELEKTRONENTOMOGRAPHIETISCHES
COMMANDE D'ÉTAGE DE TOMOGRAPHIE ÉLECTRONIQUE

(43) Date of publication of application: 04.06.2025
(73) Proprietor: FEI Company, Hillsboro, OR 97124 (US)
(72) Inventor: DE JONG, Erwin, Hillsboro (US); THEEUWES, Jeroen, Hillsboro (US); RADULESCU, Andrei, Hillsboro (US); BOER IWEMA, Reint, Hillsboro (US)
(74) Representative: Pecharová, Petra

(56) References cited:
- MASTRONARDE ET AL: "Automated electron microscope tomography using robust prediction of specimen movements", JOURNAL OF STRUCTURAL BIOLOGY, ACADEMIC PRESS, UNITED STATES, vol. 152, no. 1, 24 August 2005 (2005-08-24), pages 36 - 51, XP027192977, ISSN: 1047-8477, [retrieved on 20051001]

## Description

### Field of the Invention

The present invention relates to a system and method for generating electron tomography data and images, and in particular improving the acquisition of tilt-series data collection.

### Background of the Invention

Planar samples include structures and features on its surface and throughout the thickness of the sample. Features can interact with an electron beam and these interactions are detected and provide structural information about the samples.

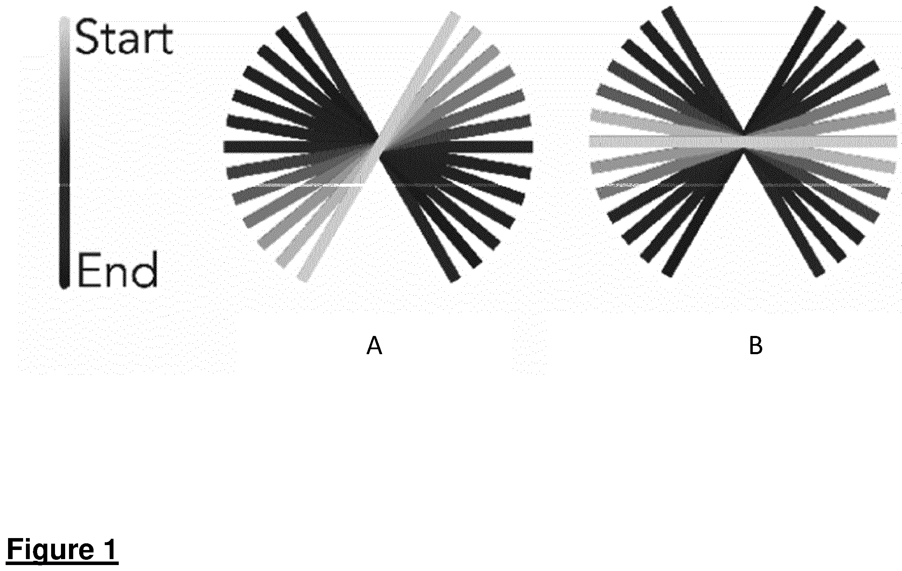

The electron beam can be directed at particular locations on and within a sample. Furthermore, the electron beam can be deflected short distances (e.g., 1-3µm) across the surface of the sample to image neighbouring features. However, in order to measure interactions with features further apart, the sample must be translated mechanically using a translation stage under the electron beam. Three-dimensional structural information can be obtained by tilting the sample in small increments (e.g., 3º) with the electron beam being detected at different tilt angles with the electron beam focused on the same feature. A tilt series of data may involve tilting the sample over ± 60º or 70º, for example. The collected data may be processed to provide an output image showing the three-dimensional structure of the feature on the sample. Figure 1 shows a schematic diagram indicating how data are collected with the sample at different tilt angles with the electron beam focussed on the same location of the sample. Figure 1A shows a continuous tilt scheme and Figure 1B shows a dose-symmetric tilt scheme.

David N. Mastronarde, "Automated electron microscope tomography using robust prediction of specimen movements", Journal of Structural Biology, Volume 152, Issue 1, 24 August 2005, Pages 36-51, ISSN 1047-8477, https://doi.org/10.1016/j.jsb.2005.07.007A, XP027192977 discloses a method for automatically obtaining electron tomography data from a sample at a series of specimen tilts. The method uses changes in specimen position at previous tilt angles to predict the position at the current tilt angle.

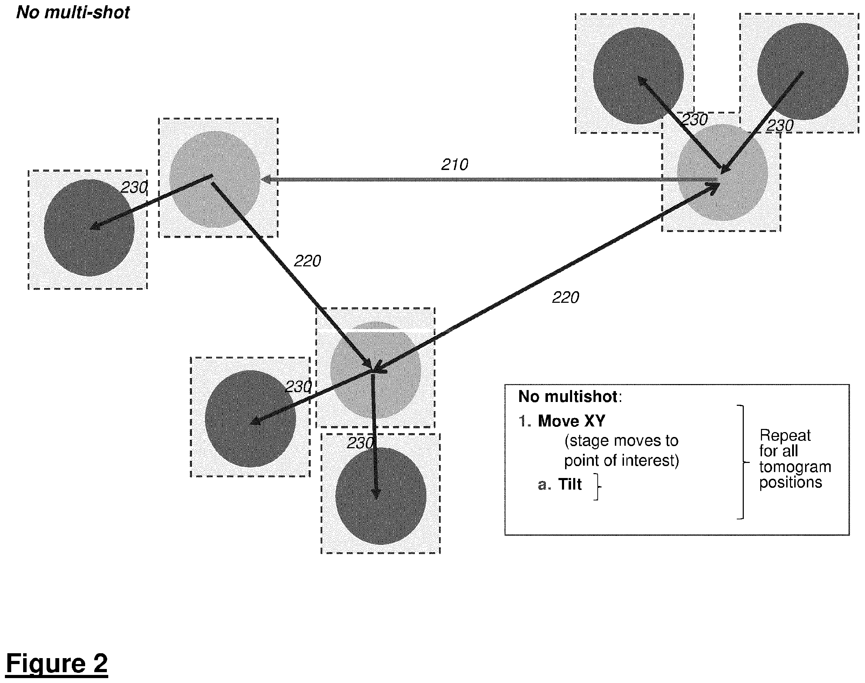

Figure 2 illustrates a different existing process for obtaining an example tilt series from eight different features on a sample. Because the features are separated by greater than the electron beam can be steered, the sample must be mechanically translated by the translation stage between each tilt series collection of data. Each time the tilted sample is translated, the electron beam needs to be refocussed. This can be achieved by a combination of moving the sample towards or away from a beam source (i.e., z-axis translation to achieve a course focus correction) and by using focussing optics with the electron beam source. Solid arrows in Figure 2 indicate mechanical translation of the sample. Arrow 230 illustrates and initial X-Y translation (e.g., perpendicular to the electron beam) at the start of an acquisition series. Arrows 220 indicate long range translation using the mechanical stage (X-Y). Arrows 220 indicate short range translation between neighbouring features using the mechanical stage. The stage moves to a point of interest. The sample is tilted about the electron beam focus point on the sample. This is repeated for all tomogram positions. Whilst effective, this data collection can be time consuming and leads to low sample throughput.

Figure 2 also shows the interaction steps for completing a tilt series with each feature investigated at different tilt angles as a legend within the figure. The mechanical translation of the sample (arrows 220) is carried out for every feature or point of interest. For every feature, a full tilt series is carried out. For example, the angle of the surface of the sample is changed by +/- 70 degrees in 3º steps with data collected at each step from the feature under the electron beam. This is repeated for all tomogram positions. These data are applied to a computed tomography algorithm to form a computed image. In the example shown in Figure 2 there are eight features being investigated with mechanical translation for each feature. Therefore, eight separate sets of data are required in this particular example, which requires a considerable amount of acquisition time.

Furthermore, whilst the sample is mainly flat, some features extend from the surface more than others and it is difficult to obtain accurate height information regarding these features.

Therefore, there is required a method and system that overcomes these problems.

### Summary of the Invention

A method and system provides efficiency improvements when collecting data to be used to generate a computed tomography image. Data are collected from points on the surface of a sample at particular different tilt angles whilst translating the sample at a current tilt angle. Focus of an electron beam is maintained (e.g., using a mechanical or piezo stage) at the sample surface, whilst being translated at the tilt angle. This avoids having to refocus the electron beam between imaging points. This can be achieved by accurately translating samples at particular tilt angles (in three dimensions) using a translation stage.

An initial step is to direct the electron beam on a first point or feature of the sample. The sample is then tilted whilst maintaining focus of the electron beam at the surface of the sample. The feature is imaged by detecting the electron beam passing through this point with data being collected and stored. The sample is translated at this tilt angle to move the electron beam so that it is directed to a second location of the surface of the sample. The electron beam is detected at this new location. These steps are repeated.

In accordance with a first aspect there is provided a method for obtaining electron tomography data from a sample (e.g., a planar sample and/or a lamella sample), the method comprising the steps of:
a) focussing an electron beam at a first location on a surface of the sample;
b) tilting the surface of the sample to a tilt angle to the electron beam while maintaining the surface of the sample at the focus of the electron beam;
c) detecting the electron beam focussed at the first location on the surface of the sample;
d) translating the sample at the tilt angle to move the focus of the electron beam to at least a second location of the surface of the sample;
e) detecting the electron beam focussed on at least the second location on the surface of the sample; and
repeating steps b) to e) at one or more different tilt angles. Therefore, data may be collected faster and more efficiently because the electron beam does not need to be refocussed on the sample surface after it has been translated to a new location or sample feature. This is because fewer tilt series are required as all rotating through all angles (i.e., alpha tilts) is more time consuming than translational X-Y-Z movement. Rather than only using lateral X-Y mechanical translation of the sample (i.e., movement in a plane perpendicular to an electron beam axis), which causes the height of a tilted sample to change relative to the electron beam and cause a defocus on the sample surface, the sample is translated in all X-Y-Z dimensions (i.e., also translated in the direction of the electron beam axis). In other words, the sample is translated so that the beam is focused on the surface of the sample at the second location, where the translation includes a component in the direction of the electron beam.

Preferably, the method may further comprise the step of generating a computed tomography (CT) image of the sample using data obtained when detecting the electron beam. Different CT algorithms may be used.

Optionally, the tilt angle may be a non-normal angle to the electron beam. The tilt angle may start normal or non-normal to an axis of the electron beam. Where the tilt angle is non-normal to the electron beam then the sample will need to be translated in three dimensions, i.e., the translation stage must move the sample same in X, Y, and Z directions. The Z component is used during this translation. Otherwise, the electron beam will not maintain focus on the sample. The X-Y plane may be considered as a plane perpendicular (normal) to the electron beam. Translating the sample so that the beam is focused on the surface of the sample at the second location, may require the translation of the sample to include a component (i.e., defined as the Z-component) in the direction of the electron beam when the tilt angle is non-normal to the electron beam.

Preferably, a first feature of the sample may be located at the first location and a second feature of the sample may be located at the second location. Therefore, multiple features within the sample may be analysed more quickly. The translation may occur during a tilt series. Each feature or a plurality of features may be investigated with the sample at the same tilt angle. Therefore, translation of the sample using the mechanical stage (one or more times) may occur at the same tilt angle with translations repeated for different tilt angles.

Optionally, the method may further comprise the steps of:
f) measuring a first distance between the first feature and the second feature normal to the electron beam with the sample tilted at the tilt angle;
g) measuring a second distance between the first feature and the second feature normal to the electron beam with the sample tilted at the one or more different tilt angles; and
h) calculating a difference in separation of the first feature and the second feature from the surface of the sample based on a difference between the first distance and the second distance and a difference between the tilt angle and the one or more different tilt angles. These additional method steps provide information regarding the height above the sample surface of different features. This additional information is obtained by stereogrammetry. The more different tilt angles that are used then the higher the accuracy of height information that can be provided. The process may be repeated at different tilt angles to improve accuracy.

Optionally, any of the tilt angle or the one or more different tilt angles may be normal to an axis of the electron beam.

Optionally, the sample is a lamella sample. However, any planar sample can be used.

Optionally, the method may further comprise the steps of:
after step c) and before step d) adjusting the electron beam to focus on a third location (or more than three locations); and
detecting the electron beam focussed at the third location on the surface of the sample. The electron beam can be deflected by small amounts (e.g., less than 3µm) without requiring translation of the sample using a mechanical stage. Therefore, features that are close to each other (e.g., a cluster of features) can be investigated more quickly (and without sacrificing optical quality) by only deflecting or moving the electron beam but at the same tilt angle. A combination of electron beam deflections and mechanical translations can be carried out at the same tilt angle during a tilt series. Therefore, this provides improved flexibility and further speeds up data acquisition. Furthermore, data acquisitions times can be reduced where there are larger distances between features without sacrificing optical quality and without a penalty on throughput.

Optionally, the method may further comprise the steps of:
after step d) and before or after step e) adjusting the electron beam to focus on a fourth location; and
detecting the electron beam focussed at the fourth location (or more than four locations) on the surface of the sample. The electron beam may be adjusted or deflected at different stages of the method.

Optionally, an angular difference between the tilt angle and the one or more different tilt angles may be between 0.1º and 10º (e.g., at or between 1º and 5º). For example, the angle between tilts may be 3º or less (e.g., 2º, 1º or less than 1º).

Optionally, the sample may be translated at the tilt angle to move the location of the electron beam on the sample by between 1µm and 3µm. Other translation distances may be used depending on where particular features are located.

According to a second aspect, there is provided an electron microscopy system comprising:
an electron beam source;
electron beam focussing optics configured to focus the electron beam on a surface of a sample;
a sample translation stage configured to translate the sample in a plane normal to the electron beam and vary a tilt angle of the surface of the sample relative to an axis of the electron beam; and
a control unit in communication with the sample translation stage and the electron beam focussing optics and configured to carry out the steps of:
   a) focus the electron beam at a first location on the surface of the sample;
   b) tilt the surface of the sample to a tilt angle to the electron beam while maintaining the surface of the sample at the focus of the electron beam;
   c) detect the electron beam focussed at the first location on the surface of the sample;
   d) translate the sample at the tilt angle to move the focus of the electron beam to at least a second location of the surface of the sample;
   e) detect the electron beam focussed on at least the second location on the surface of the sample; and
   repeat steps b) to e) at a one or more different tilt angles.

Preferably, the electron microscopy system may further comprise:
a processor; and
memory storing executable instructions which, when executed by the processor, configure the electron microscopy system or a controller of the electron microscopy system such as a workstation, server or external computer to perform:
   generating a computed tomography image of the sample using data obtained when detecting the electron beam. This functionality may also be provided by a computer system external to the electron microscopy system.

Optionally, the control unit may be further configured to:
f) measure a first distance between the first feature and the second feature normal to the electron beam with the sample tilted at the tilt angle;
g) measure a second distance between the first feature and the second feature normal to the electron beam with the sample tilted at the one or more different tilt angles; and
h) calculate a difference in separation of the first feature and the second feature from the surface of the sample based on a difference between the first distance and the second distance and a difference between the tilt angle and the one or more different tilt angles.

Optionally, the sample translation stage may further comprise a plurality of electric motors or other actuators.

The methods described above may be implemented as a computer program comprising program instructions to operate a computer. The computer program may be stored on a computer-readable medium, including a non-transitory computer-readable medium.

The computer system may include a processor or processors (e.g. local, virtual or cloud-based) such as a Central Processing Unit (CPU), and/or a single or a collection of Graphics Processing Units (GPUs). The processor may execute logic in the form of a software program. The computer system may include a memory including volatile and non-volatile storage medium. A computer-readable medium (CRM) may be included to store the logic or program instructions. For example, embodiments may include a non-transitory CRM storing software comprising instructions executable by one or more computers which, upon such execution, cause the one or more computers to perform the disclosed methods. Non-transitory CRM may refer to a CRM that stores data for short periods or in the presence of power such as a memory device or Random Access Memory (RAM). For example, a non-transitory computer-readable medium may include storage components, such as, a hard disk (e.g., a magnetic disk, an optical disk, a magneto-optic disk, and/or a solid state disk), a compact disc (CD), a digital versatile disc (DVD), a floppy disk, a cartridge, and/or a magnetic tape. The different parts of the system may be connected using a network (e.g. wireless networks and wired networks). The computer system may include one or more interfaces. The computer system may contain a suitable operating system such as UNIX, Windows (RTM) or Linux, for example.

It should be noted that any feature described above may be used with any particular aspect or embodiment of the invention.

### Brief description of the Figures

The present invention may be put into practice in a number of ways and embodiments will now be described by way of example only and with reference to the accompanying drawings, in which:
Fig. 1 shows a schematic diagram illustrating two schemes (A and B) for obtaining electron beam tomography data;
Fig. 2 shows a schematic diagram illustrating how a sample is moved under an electron beam of an electron beam microscope;
Fig. 3 shows a schematic diagram of a computer system controlling a transmission electron microscope (TEM);
Fig. 4 shows images A and B of regions of a sample illustrating different imaging techniques;
Fig. 5 shows a schematic diagram illustrating a tilting process of a sample according to an existing imaging technique;
Fig. 6 shows a schematic diagram illustrating an imaging technique according to an example implementation;
Fig. 7 shows a schematic diagram of features on a sample illustrating translation of a sample and movement of an electron beam;
Fig. 8 shows a schematic diagram of features on a sample illustrating the translation of a sample under an electron beam according to an example implementation;
Fig. 9 shows a schematic diagram of features of a sample illustrating the translation of the sample and movement of an electron beam across the sample according to a further example implementation;
Fig. 10 shows a schematic diagram of a sample illustrating a process for obtaining height information from a sample;
Fig. 11 shows a graph illustrating a comparison of acquisition times for different imaging techniques;
Fig. 12 shows a flowchart of a method for operating the system of Figure 3 to obtain a computer tomography image of a sample;
Fig. 13 shows components of a translation stage of the TEM system of Figure 3;
Fig. 14 shows components of a translation stage of the TEM system of Figure 3; and
Fig. 15 shows components of a translation stage of the TEM system of Figure 3.

It should be noted that the figures are illustrated for simplicity and are not necessarily drawn to scale. Like features are provided with the same reference numerals.

### Detailed description of the preferred embodiments

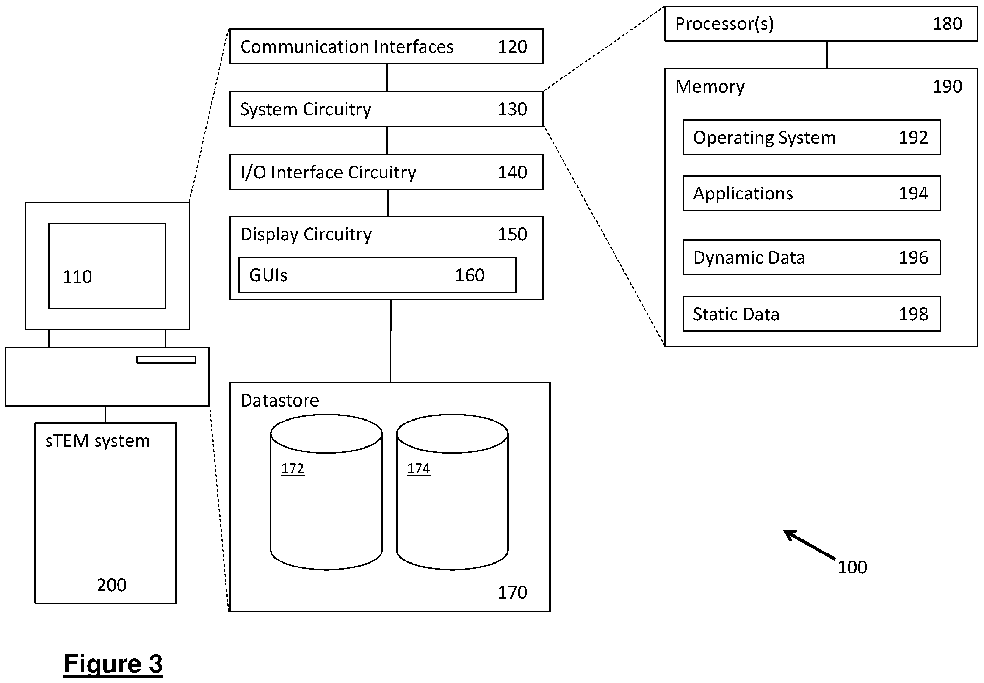

Figure 3 shows a system 100 for implementing electron beam computed tomography imaging processes described within this description. The system 100 includes a transmission electron microscope (TEM) system 200 and a computer system 110, which itself includes a number of components including communication interfaces 120, system circuitry 130, input/output (I/O) circuitry 140, display circuitry and interfaces 150, and a datastore 170. The system circuitry 120 can include one or more processors or CPUs 180 and memory 190. The system circuitry 130 may include any combination of hardware, software, firmware, and/or other circuitry. The system circuitry 130 may be implemented, with one or more systems on a chip (SoC), application specific integrated circuits (ASIC), microprocessors, and/or analog and digital circuits. The computer system 100 may also be located within the TEM system 200 or connected to it by cables or a computer network.

The display circuitry may provide one or more graphical user interfaces (GUIs) 160 and the I/O interface circuitry 140 may include touch sensitive or non-touch displays, sound, voice or other recognition inputs, buttons, switches, speakers, sounders, and other user interface elements. The I/O interface circuitry 140 may include microphones, cameras, headset and microphone input /output connectors, Universal Serial Bus (USB) connectors, and SD or other memory card sockets. The I/O interface circuitry 140 may further include data media interfaces (e.g., a CD-ROM or DVD drive) and other bus and display interfaces.

The memory 190 may include volatile (RAM) or non-volatile memory (e.g., ROM or Flash memory). The memory may store the operating system 192 of the computer system 100, applications or software 194, dynamic data 196, and/or static data 198. The datastore or data source 170 may include one or more databases 172, 174 and/or a file store or file system, for example.

The method and system may be implemented in hardware, software, or a combination of hardware and software. The method and system may be implemented either as a server comprising a single computer system or as a distributed network of servers connected across a network. Any kind of computer system or other electronic apparatus may be adapted to carry out the described methods.
In the TEM system 200, an electron beam may be deflected (e.g., using electromagnetic deflectors) across a sample and this can be achieved with great precision and repeatability. Furthermore, during such a beam deflection, focus of the electron beam on the surface of the sample may be maintained to a high degree of reliability. In order to investigate features of the sample that are further away and beyond the deflection range, the sample may be translated mechanically under the electron beam and this achieved using a mechanical translation stage. In the following description "multishot" describes a process where the translation stage moves the sample to a cluster of features. Where features in the cluster are closer than around 3 µm then the electron beam is deflected to investigate each feature in turn without requiring a mechanical translation of the sample. Where no multishot is used then the sample is mechanically translated so that each feature in turn is placed under the electron beam, which remains static (see Figure 1). In any case, a highly accurate and repeatable translation stage improves acquisition of data because this reduces the amount of refocus required of the electron beam on the sample surface. An example of a suitable mechanical translation stage is the Smart Stage by Thermo Fisher Scientific. Preferably, positioning accuracy for the translation stage for small moves (up to 500nm), reproducibility should also be of the order of 10nm for small moves (less than 500nm) and up to 50nm for large moves of several micrometres.

In order to generate a computer tomography image using electron beam signals, each feature must be repeatedly analysed at different tilt angles. In existing techniques, this is achieved by translating the sample using the translation stage to locate a feature and electron beam, tilting the sample in small angle steps (e.g., 3°) about the feature so that focus of the electron beam is maintained between tilt steps on the feature on the surface of the sample, and processing the resulting data to form a computer tomography image of the feature. Once a full tilt series of a feature is obtained then the translation stage returns the sample so that its surface is perpendicular to the beam axis and the sample is translated so that a new feature is under the electron beam with the process repeated. Suitable software for generating images from the data include Tomography 5 and Tomo Live both provided by Thermo Fisher Scientific.

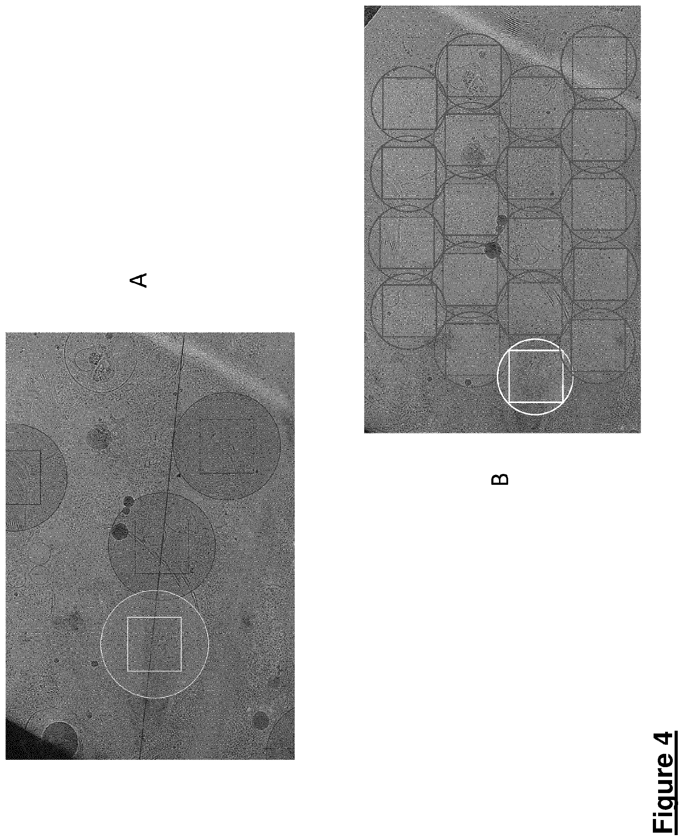

Whilst this may be automated to some extent, each time the lamella sample is translated perpendicular to the electron beam axis (X-Y translation) the electron beam may need to be refocused. This is illustrated in Figure 4A, which shows an image of a sample with the circles indicating areas containing features that are investigated between mechanical translations of the sample. The diameter of the circles is the size of the electron beam. For example, the diameter may be less than 1µm or increased in size.

Figure 4B shows an example implementation of an improved approach where multiple positions on the sample may be reached in a single tilt series with fewer optical aberrations. This improved process is described with regards to the following figures.

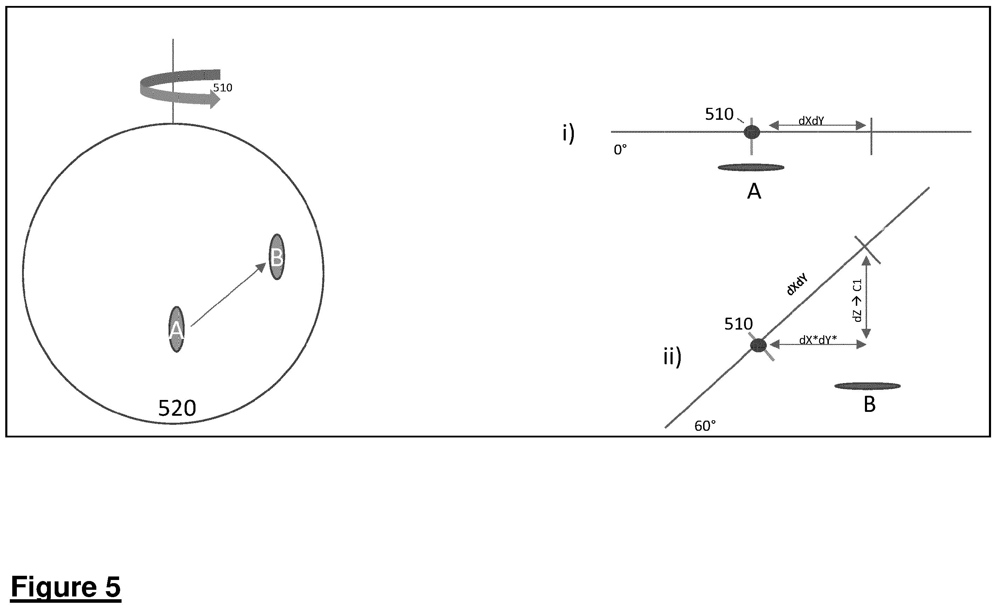

Figure 5 illustrates a current process for obtaining computer tomography data from a sample. Region 520 in Figure 5 corresponds to a multi-micron area on the sample including clusters A and B. Each cluster A and B can contain multiple features. The surface of the sample may be tilted through axis 510, which passes through cluster A. However, the electron beam cannot be deflected beyond the region of the clusters shown in region A. In order to investigate the features within cluster B then mechanical translation of the sample is required. Diagram i) in this figure illustrates translation of the sample across the plane of the sample with a tilt angle of 0°. In this case, a translation dXdY is required to bring the electron beam over cluster B.

With this configuration, no significant refocusing of the electron beam may be required as feature B remains substantially the same (Z) distance from the source of the electron beam with a 0° tilt angle. However, diagram ii shows the effect of translating the sample within the same tilt angle using the same tilt axis 510. As can be seen from diagram ii), when the electron beam is above cluster B then there will be a height (dZ) difference apparent between cluster A and cluster B. Therefore, during the same tilt series with a non-zero tilt (60° in this example), then refocusing of the electron beam will be required (e.g., by varying the electromagnetic properties of the TEM until focus is achieved) within the same tilt sequence using the same tilt axis 510. This increases the time taken to acquire data from multiple clusters in the sample.

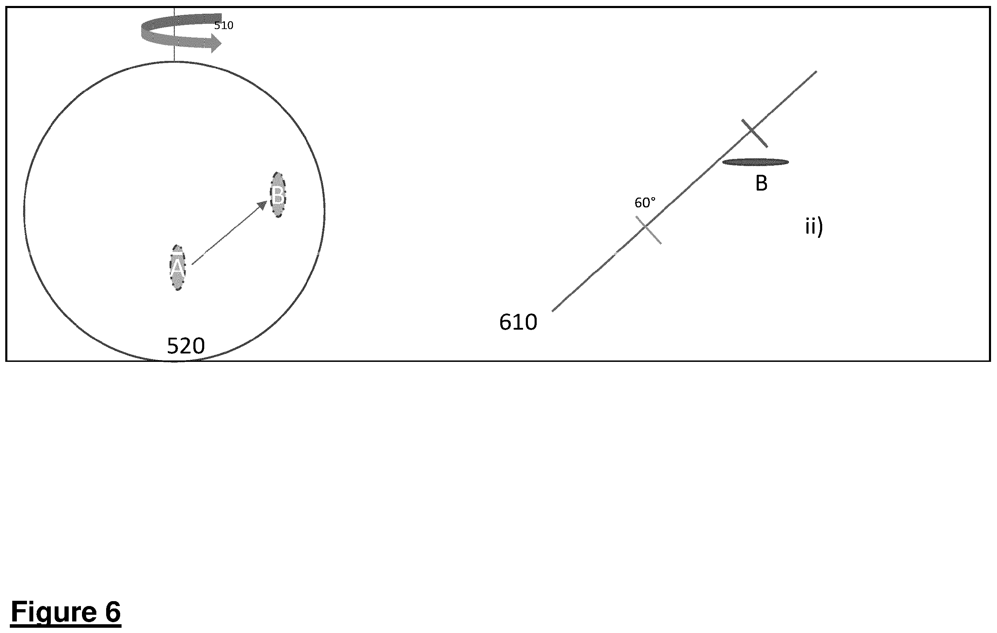

Figure 6 shows an example implementation of an improved method for acquiring data within the same tilt sequence. Again, the same sample is used with clusters A and B and a single tilt series across axis 510 on the sample. However, when translating the stage so that the new feature cluster B is under the electron beam, instead of translating the sample perpendicular to the electron beam (only using X-Y translation), the sample is translated in the plane of the tilt angle (in this example at 60°), along line 610 in the figure. Therefore, when moving between clusters on the sample, focus of the electron beam is substantially maintained at the surface of the sample resulting in faster data acquisition. This results in fewer aberrations and the entire sample can be reached using X, Y and Z changes using the mechanical translation stage.

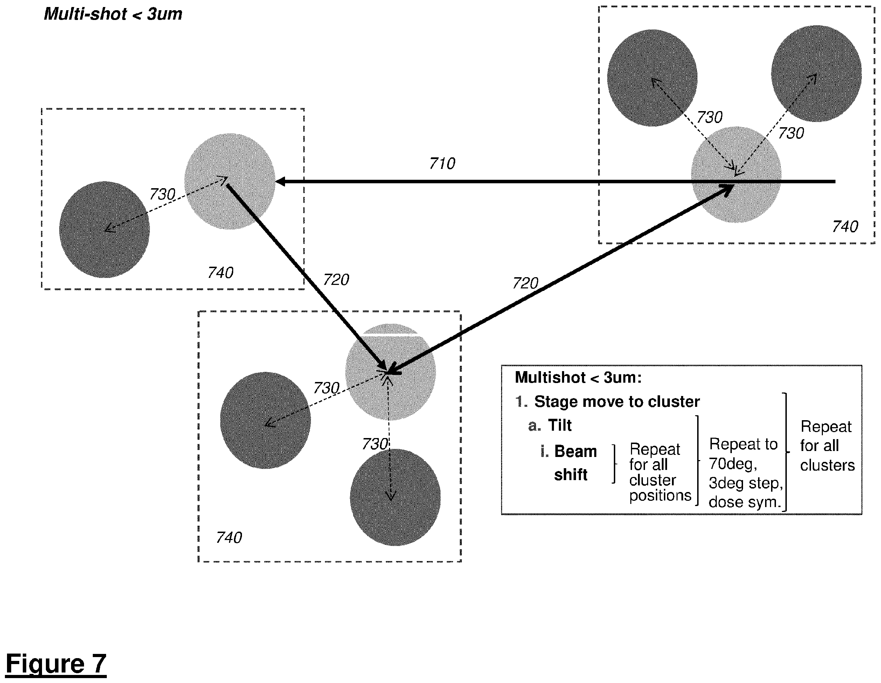

Figure 7 illustrates a further existing implementation showing the different steps and iterations used to obtain computer tomography information from a sample. This process may be described with regards to the process shown in Figure 2. Arrow 710 indicates an initial X-Y translation to a starting position on the sample. In the process illustrated by Figure 7, three tilt sequences are carried out with individual clusters shown bounded by dotted lines. Within each cluster, deflection of the electron beam is indicated by dotted arrows 730 and mechanical translations between clusters are indicated by solid arrows 720. Therefore, this process may be described as a multishot acquisition with each cluster containing features within a deflection range of the electron beam. The legend within Figure 7 shows the data acquisition iterations.

The stage is moved to a first cluster or initial starting point (arrow 710). A full tilt series is carried out within each cluster region (740). Data is acquired from each feature in the cluster at the same tilt angle with the electron beam deflected (arrow 730) with the sample at the same tilt angle in the tilt series. This is repeated for all positions in a particular cluster (i.e. each feature in the cluster) so that the sample is tilted to a new tilt position (e.g. +/- 3°). No mechanical translation is carried out within the same tilt series. Overall, this process is repeated for clusters with a mechanical translation between clusters (arrow 720) but electron beam focussing is required whenever a mechanical translation (between cluster regions 740) takes place. Translation of the sample is in the X-Y plane only so refocusing of the electron beam is required at each different cluster following the X-Y translations. This is for the reasons provided with regards to Figure 5. Whilst the use of multishot can reduce the time taken to acquire data, the need to refocus between each cluster has drawbacks.

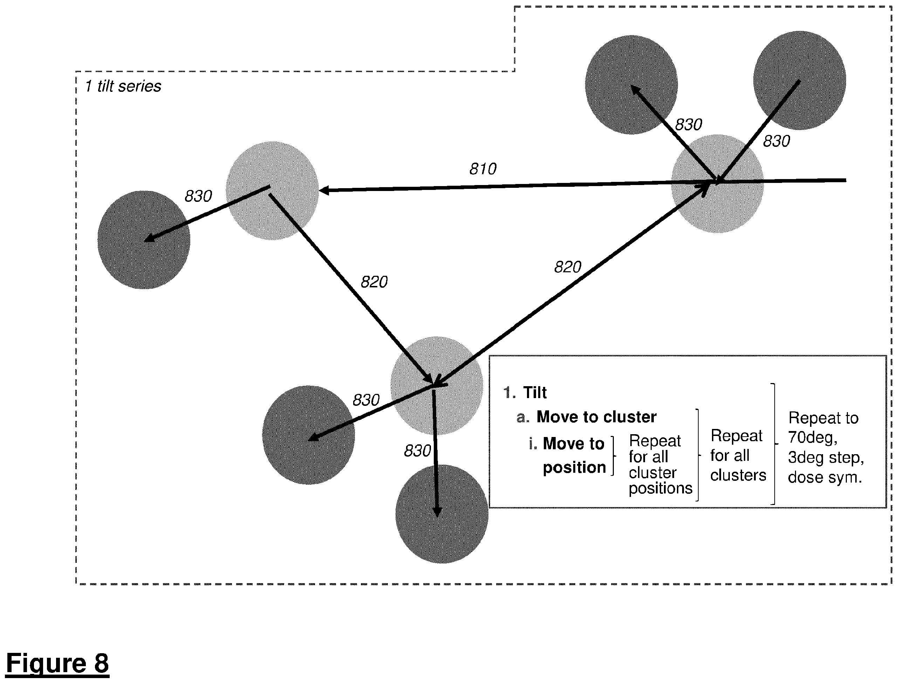

Figure 8 shows an illustrative example according to the improved method for obtaining computed tomography electron beam data. In this case, only a single tilt series is required for the entire sample of all clusters, even when mechanical translation is required beyond the range of electron beam deflection. As in the previous figures, the dotted lines indicate deflection of the electron beam between features in an individual cluster. Solid lines of arrows indicate mechanical translation of the sample. In the example process shown in Figure 8, only mechanical translations are used. However, when the sample is translated, it is moved according to the process described with respect to Figure 6. That is, the sample is translated in the plane of the surface of the sample whatever its tilt angle rather than being restricted to the X-Y plane. In other words, a Z component is included to maintain focus of the electron beam on the surface of the sample when it is mechanically translated.

Therefore, each feature in every cluster can be investigated using the electron beam at a single tilt position and so a single tilt series is required for all feature in all clusters of the sample. As shown in Figure 8, the translation stage moves between clusters and also within clusters whilst maintaining focus of the electron beam on the surface of the sample.

Again, the legend within Figure 8 shows the steps within each tilt iteration. For a single angle, the translation stage moves to a particular cluster, moves to a position within that cluster and acquires data at each position. Once each feature in every cluster has been imaged then the tilt angle is changed and the process is repeated for a different tilt angle. No significant refocussing of the electron beam is required following mechanical translation.

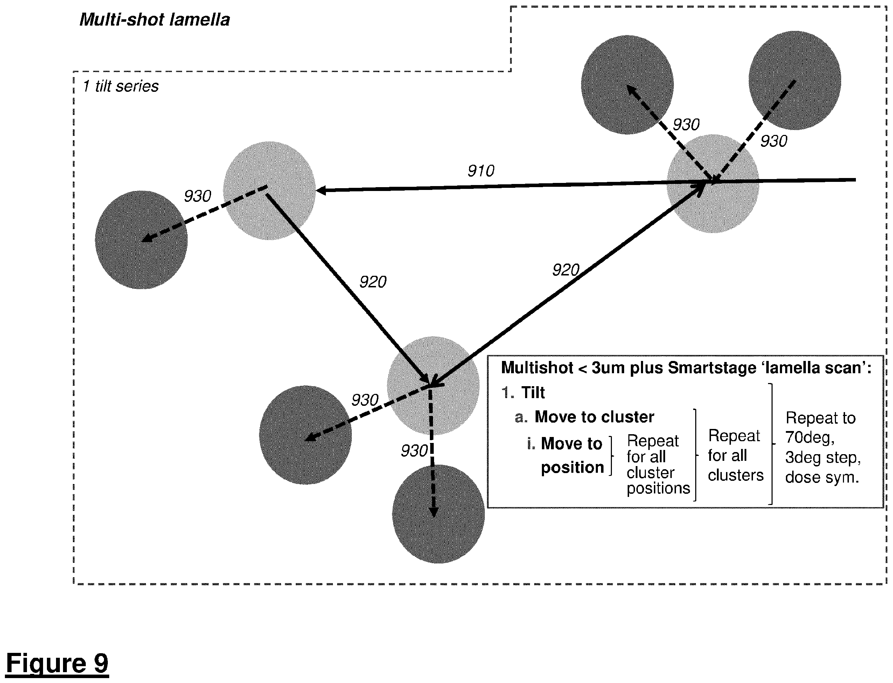

Figure 9 shows a similar process to that described with reference to Figure 8. However, in Figure 9 instead of the mechanical translation of the to investigate features within individual clusters, the electron beam is deflected (see dotted arrows 930), which further reduces acquisition time as this deflection can be faster than an X-Y-Z translation of the sample. Overall, this can achieve up to 30% improvement in speed with regards to existing multishot techniques. Mechanical translation between clusters (arrows 920) is still carried out within the same tilt series.

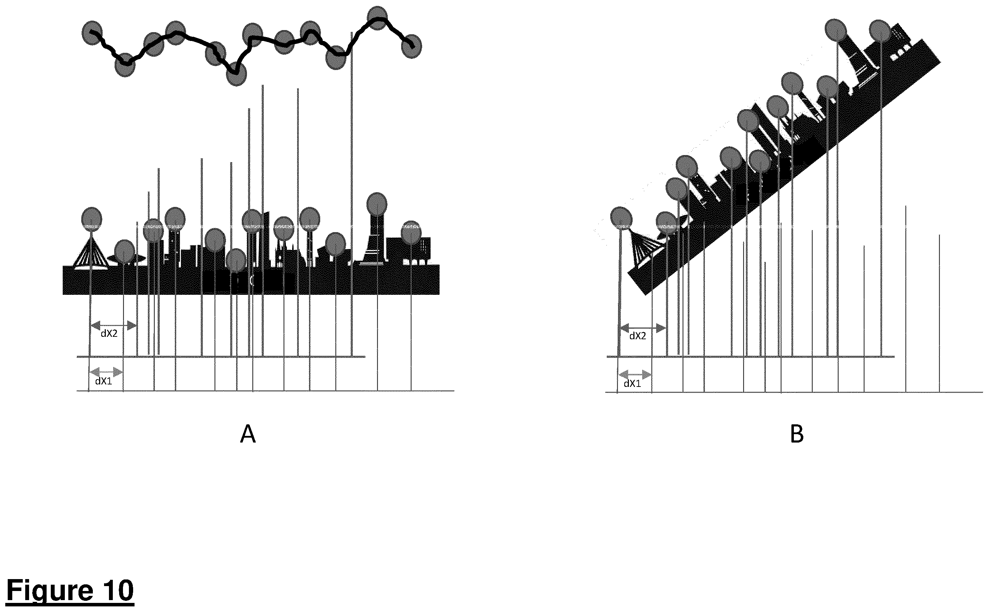

Figure 10 shows a schematic diagram of a sample in different configurations (A and B). However, in this example, features on the sample have different heights relative to each other above the surface of the sample.

Height information from the different features may be obtained at the same time as carrying out the previously described tilt series or as a separate process. Existing techniques may obtain height information using just tilt angle and measured distance between features but with limited accuracy. An improved approach to obtaining a more accurate estimate of feature heights may be achieved by comparing distances in measured and estimated distance between points at two or more different angles. In the diagrams A and B of Figure 10, dX1 shows the apparent distance between features with the sample perpendicular to the electron beam axis and dX2 is the apparent distance between the features on the sample when tilted at an angle normal to the electron beam axis (e.g., during the previously described tilt series). Due to this tilt angle, dX1 is not equal to dX2 (i.e., in the X-Y plane). The delta or difference between dX1 and dX2 is a measure of height or difference between the top of the feature and the surface of the sample with the larger of the change in height causing a larger delta between dX1 and dX2. This can be determined as function of alpha tilt. When repeated at different angles then this estimation of height can be improved further, e.g., using trigonometric analysis incorporating the known tilt angles.

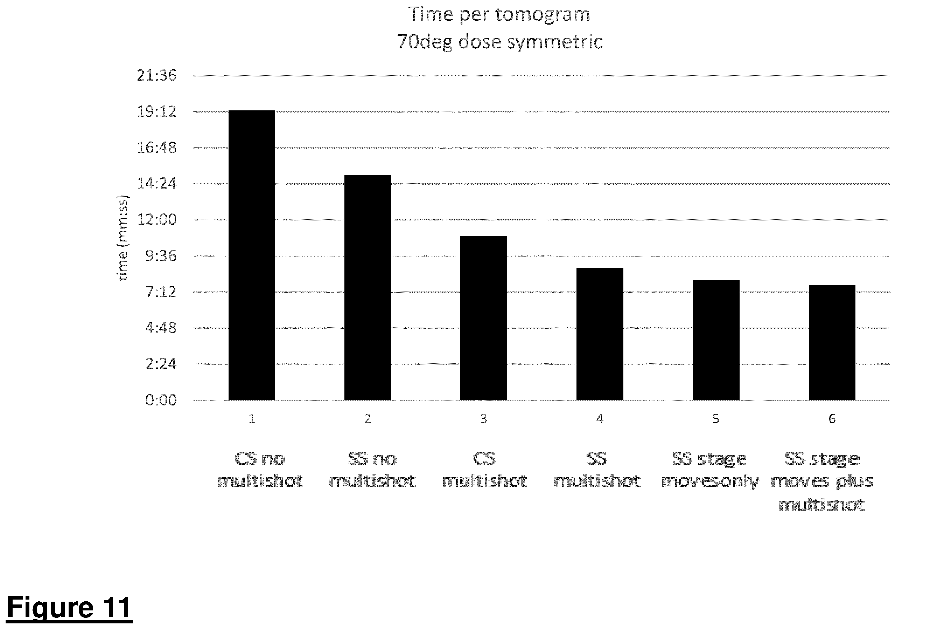

Figure 11 shows example results between different existing schemes for obtaining tilt series computed tomography electron data and the improved methods described throughout this description. as mentioned previously, these improvements may reduce the time taken to acquire a full tilt series by around 30%.

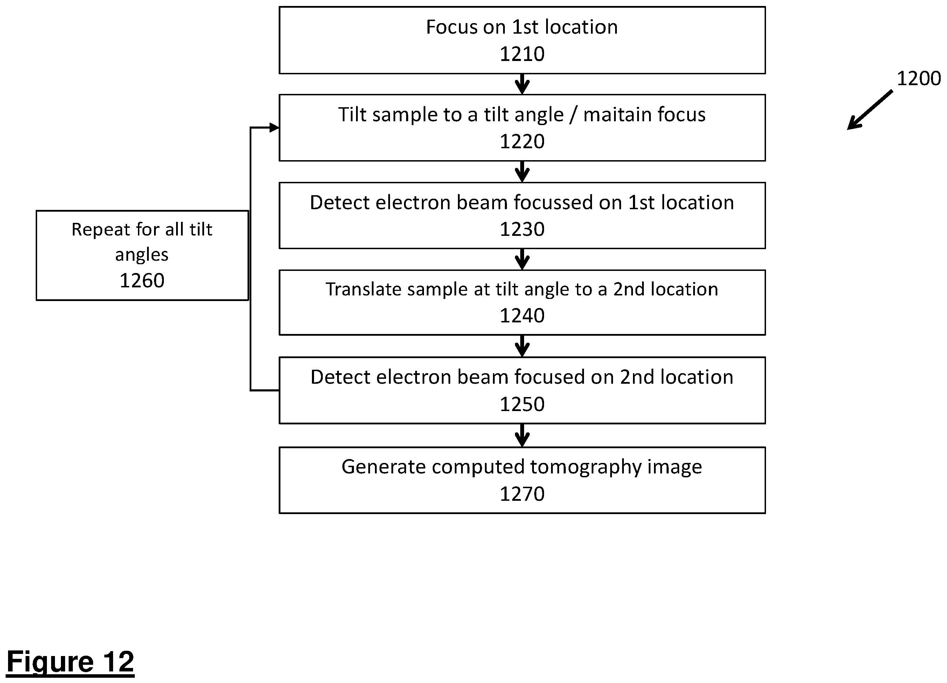

Figure 12 shows a flowchart of the method 1200 for obtaining electron tomography data from a sample. At step 1210, the electron beam is focused at a first location on the surface of the sample. At step 1220, the surface of the sample is tilted to an angle to the electron beam while maintaining the surface of the sample at the focus of the electron beam.

At step 1230, the electron beam is detected when it is focused at the first location of the surface of the sample. At step 1240, the sample is translated at the tilt angle (i.e., in an X, Y, Z plane) to move the focus of the electron beam to at least a second location (or further locations) on the surface of the sample. At step 1250, the electron beam is detected when focused on the second location on the surface of the sample. Step 1260 indicates that this is repeated for all tilt angles in the tilt series (e.g., for two or more features).

Once all of the data from all of the features have been collected then a computer tomography image is generated at step 1270.

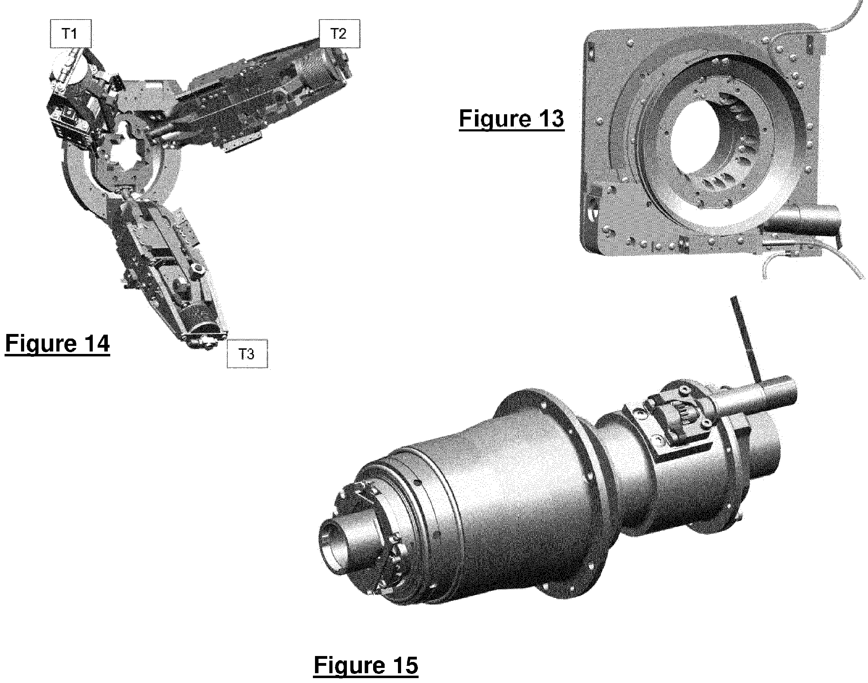

As described previously, accurate mechanical translation can improve data acquisition times provided that the sample is translated at the same angle as the tilt angle applied to the sample. Figures 13, 14 and 15 show components of an example translation stage that can achieve this (e.g., the SmartStage device). The translation stage provides a translation of the sample in three dimensions (X, Y and Z), which is stacked on the alpha tilt.

Rx may be described as alpha tilt and may be realized using a (stiff) bearing with a worm wheel (see Figure 13). The worm wheel is driven by a worm on a DC motor. The Rx position is measured on the DC motor. There are two bearings stacked on top of each other in this example mechanism. A so called "dirty" bearing for the driving torque and a "clean" bearing where no or very low forces are applied. On the clean bearing a second sensor is placed which measures rotation using an encoder. There is friction and play in the drivetrain, but due to the second sensor which directly measures sample rotation, the exact sample Rx is known and can be controlled more accurately.

The translation stage may be friction free for XYZ translations. Position may be measured at a location where there is only stiffness towards the sample position with no friction or play. Preferably, only elastic components are used in the translation stage.

There are three (similar) drive units (T1, T2 and T3 in Figure 14) which are placed in a tripod construction with a pivot point in the front. A tilt tube is used which is moved in XYZ directions at the rear of each arm (see Figure 15). A holder is placed inside the tilt tube but does not move with respect to this tube. Using a pivot point in the front consisting of leaf springs, the XYZ position of the sample may be controlled and measured. Because there is no friction from the measurement position to the sample position, play can be reduced or eliminated and the sample position can be exactly controlled, leading to repeatability and accuracy in the order of 10s of nms. Other suitable translation stages may be used that can accurately control movement in three dimensions.

As used throughout, including in the claims, unless the context indicates otherwise, singular forms of the terms herein are to be construed as including the plural form and vice versa. For instance, unless the context indicates otherwise, a singular reference herein including in the claims, such as "a" or "an" (such as an ion multipole device) means "one or more" (for instance, one or more ion multipole device). Throughout the description and claims of this disclosure, the words "comprise", "including", "having" and "contain" and variations of the words, for example "comprising" and "comprises" or similar, mean "including but not limited to", and are not intended to (and do not) exclude other components. Also, the use of "or" is inclusive, such that the phrase "A or B" is true when "A" is true, "B is true", or both "A" and "B" are true.

The use of any and all examples, or exemplary language ("for instance", "such as", "for example" and like language) provided herein, is intended merely to better illustrate the disclosure and does not indicate a limitation on the scope of the disclosure unless otherwise claimed. No language in the specification should be construed as indicating any non-claimed element as essential to the practice of the disclosure.

The terms "first" and "second" may be reversed without changing the scope of the disclosure. That is, an element termed a "first" element may instead be termed a "second" element and an element termed a "second" element may instead be considered a "first" element.

Any steps described in this specification may be performed in any order or simultaneously unless stated or the context requires otherwise. Moreover, where a step is described as being performed after a step, this does not preclude intervening steps being performed.

It is also to be understood that, for any given component or embodiment described throughout, any of the possible candidates or alternatives listed for that component may generally be used individually or in combination with one another, unless implicitly or explicitly understood or stated otherwise. It will be understood that any list of such candidates or alternatives is merely illustrative, not limiting, unless implicitly or explicitly understood or stated otherwise.

Unless otherwise described, all technical and scientific terms used throughout have a meaning as is commonly understood by one of ordinary skill in the art to which the various embodiments described herein belongs.

As will be appreciated by the skilled person, details of the above embodiment may be varied without departing from the scope of the present invention, as defined by the appended claims.

For example, a different translation stage may be used. The microscope may also be an energy filtered transmission electron microscope (EFTEM) or a scanning TEM, for example.

The term "focus" in this disclosure is used to describe directing the electron beam to achieve a particular required set of beam properties when intersecting a feature, for example. These properties may include beam or spot size (e.g., diameter or circular diameter), beam or spot shape (e.g., a circle), intensity, beam uniformity (e.g., variation in intensity across the beam or spot), and/or beam direction, as the electron beam passes through a particular plane, such as a sample plane. Therefore, focussing the electron beam may also mean forming or directing the electron beam so that it has particular properties at a particular plane or location. "Focus" or "focussing" may be used interchangeably with "direct", "directing", "detect", "detecting", "image", or "imaging".

## Claims

1. A method for obtaining electron tomography data from a sample, the method comprising the steps of:
a) focussing an electron beam at a first location on a surface of the sample;
b) tilting the surface of the sample to a tilt angle to the electron beam while maintaining the surface of the sample at the focus of the electron beam;
c) detecting the electron beam focussed at the first location on the surface of the sample;
d) translating the sample at the tilt angle to move the focus of the electron beam to at least a second location of the surface of the sample;
e) detecting the electron beam focussed on at least the second location on the surface of the sample; and
repeating steps b) to e) at one or more different tilt angles.

2. The method of claim 1, further comprising the step of generating a computed tomography image of the sample using data obtained when detecting the electron beam.

3. The method according to any previous claim wherein the tilt angle is a non-normal angle to the electron beam.

4. The method of any previous claim, wherein a first feature of the sample is located at the first location and a second feature of the sample is located at the second location.

5. The method of claim 4 further comprising the steps of:
f) measuring a first distance between the first feature and the second feature normal to the electron beam with the sample tilted at the tilt angle;
g) measuring a second distance between the first feature and the second feature normal to the electron beam with the sample tilted at the one or more different tilt angles; and
h) calculating a difference in separation of the first feature and the second feature from the surface of the sample based on a difference between the first distance and the second distance and a difference between the tilt angle and the one or more different tilt angles.

6. The method of claim 5, wherein any of the tilt angle or the one or more different tilt angles is normal to an axis of the electron beam.

7. The method according to any previous claim, wherein the sample is a lamella sample.

8. The method according to any previous claim further comprising the steps of:
after step c) and before step d) adjusting the electron beam to focus on a third location; and
detecting the electron beam focussed at the third location on the surface of the sample.

9. The method according to any previous claim further comprising the steps of:
after step d) and before or after step e) adjusting the electron beam to focus on a fourth location; and
detecting the electron beam focussed at the fourth location on the surface of the sample.

10. The method according to any previous claim, wherein an angular difference between the tilt angle and the one or more different tilt angles is between 0.1 and 10 degrees.

11. The method according to any previous claim, wherein the sample is translated at the tilt angle to move the location of the electron beam on the sample by between 1µm and 3µm.

12. An electron microscopy system comprising:
an electron beam source;
electron beam focussing optics configured to focus the electron beam on a surface of a sample;
a sample translation stage configured to translate the sample in a plane normal to the electron beam and vary a tilt angle of the surface of the sample relative to an axis of the electron beam; and **characterised by**
a control unit in communication with the sample translation stage and the electron beam focussing optics and configured to carry out the steps of:
a) focus the electron beam at a first location on the surface of the sample;
b) tilt the surface of the sample to a tilt angle to the electron beam while maintaining the surface of the sample at the focus of the electron beam;
c) detect the electron beam focussed at the first location on the surface of the sample;
d) translate the sample at the tilt angle to move the focus of the electron beam to at least a second location of the surface of the sample;
e) detect the electron beam focussed on at least the second location on the surface of the sample; and
repeat steps b) to e) at a one or more different tilt angles.

13. The electron microscopy system of claim 12 further comprising:
a processor; and
memory storing executable instructions which, when executed by the processor, configure the electron microscopy system to perform:
generating a computed tomography image of the sample using data obtained when detecting the electron beam.

14. The electron microscopy system of claim 13, wherein the control unit is further configured to:
f) measure a first distance between the first feature and the second feature normal to the electron beam with the sample tilted at the tilt angle;
g) measure a second distance between the first feature and the second feature normal to the electron beam with the sample tilted at the one or more different tilt angles; and
h) calculate a difference in separation of the first feature and the second feature from the surface of the sample based on a difference between the first distance and the second distance and a difference between the tilt angle and the one or more different tilt angles.

15. The electron microscopy system of claim 13 or claim 14, wherein the sample translation stage further comprises a plurality of electric motors.

## Patentansprüche

1. Verfahren zum Erhalten von Elektronentomographie-Daten von einer Probe, wobei das Verfahren die folgenden Schritte umfasst:
a) Fokussieren eines Elektronenstrahls an einer ersten Stelle auf einer Fläche der Probe;
b) Neigen der Fläche der Probe in einem Neigungswinkel zu dem Elektronenstrahl, während die Fläche der Probe im Fokus des Elektronenstrahls gehalten wird;
c) Detektieren des an der ersten Stelle auf der Fläche der Probe fokussierten Elektronenstrahls;
d) Verschieben der Probe in dem Neigungswinkel, um den Fokus des Elektronenstrahls zu mindestens einer zweiten Stelle auf der Fläche der Probe zu bewegen;
e) Detektieren des an mindestens der zweiten Stelle auf der Fläche der Probe fokussierten Elektronenstrahls; und
Wiederholen der Schritte b) bis e) bei einem oder mehreren unterschiedlichen Neigungswinkeln.

2. Verfahren nach Anspruch 1, ferner umfassend den Schritt des Erzeugens eines Computertomographie-Bildes der Probe unter Verwendung der Daten, die beim Detektieren des Elektronenstrahls erhalten werden.

3. Verfahren nach einem der vorhergehenden Ansprüche, wobei der Neigungswinkel ein nicht-senkrechter Winkel zum Elektronenstrahl ist.

4. Verfahren nach einem der vorhergehenden Ansprüche, wobei sich ein erstes Merkmal der Probe an der ersten Stelle befindet und sich ein zweites Merkmal der Probe an der zweiten Stelle befindet.

5. Verfahren nach Anspruch 4, ferner umfassend die folgenden Schritte:
f) Messen eines ersten Abstands zwischen dem ersten Merkmal und dem zweiten Merkmal senkrecht zum Elektronenstrahl, wobei die Probe in dem Neigungswinkel geneigt ist;
g) Messen eines zweiten Abstands zwischen dem ersten Merkmal und dem zweiten Merkmal senkrecht zum Elektronenstrahl, wobei die Probe in dem einen oder den mehreren unterschiedlichen Neigungswinkeln geneigt ist;
und
h) Berechnen einer Differenz im Abstand des ersten Merkmals und des zweiten Merkmals von der Fläche der Probe auf Grundlage einer Differenz zwischen dem ersten Abstand und dem zweiten Abstand und einer Differenz zwischen dem Neigungswinkel und dem einen oder den mehreren unterschiedlichen Neigungswinkeln.

6. Verfahren nach Anspruch 5, wobei der Neigungswinkel oder der eine oder die mehreren unterschiedlichen Neigungswinkel senkrecht zu einer Achse des Elektronenstrahls ist/sind.

7. Verfahren nach einem der vorhergehenden Ansprüche, wobei es sich bei der Probe um eine Lamellenprobe handelt.

8. Verfahren nach einem der vorhergehenden Ansprüche, ferner umfassend die folgenden Schritte:
Justieren des Elektronenstrahls, um auf eine dritte Stelle zu fokussieren, nach Schritt c) und vor Schritt d); und Detektieren des an der dritten Stelle auf der Fläche der Probe fokussierten Elektronenstrahls.

9. Verfahren nach einem der vorhergehenden Ansprüche, ferner umfassend die folgenden Schritte:
Justieren des Elektronenstrahls, um auf eine vierte Stelle zu fokussieren, nach Schritt d) und vor Schritt e); und
Detektieren des an der vierten Stelle auf der Fläche der Probe fokussierten Elektronenstrahls.

10. Verfahren nach einem der vorhergehenden Ansprüche, wobei eine Winkeldifferenz
zwischen dem Neigungswinkel und dem einen oder den mehreren unterschiedlichen Neigungswinkeln zwischen 0,1 und 10 Grad beträgt.

11. Verfahren nach einem der vorhergehenden Ansprüche, wobei die Probe in dem Neigungswinkel verschoben wird, um die Stelle des Elektronenstrahls auf der Probe um 1 µm bis 3 µm zu verschieben.

12. Elektronenmikroskopiesystem, umfassend:
eine Elektronenstrahlquelle;
Elektronenstrahl-Fokussierungsoptik, die konfiguriert ist, um den Elektronenstrahl auf der Fläche einer Probe fokussieren;
einen Probenverschiebungstisch, der konfiguriert ist, um die Probe in einer Ebene senkrecht zum Elektronenstrahl zu verschieben und einen Neigungswinkel der Fläche der Probe relativ zu einer Achse des Elektronenstrahls zu variieren; und **gekennzeichnet durch**
eine Steuereinheit in Kommunikation mit dem Probenverschiebungstisch und der Elektronenstrahl-Fokussierungsoptik, die zum Durchführen der folgenden Schritte konfiguriert ist:
a) Fokussieren des Elektronenstrahls an einer ersten Stelle auf der Fläche der Probe;
b) Neigen der Fläche der Probe in einem Neigungswinkel zu dem Elektronenstrahl, während die Fläche der Probe im Fokus des Elektronenstrahls gehalten wird;
c) Detektieren des an der ersten Stelle auf der Fläche der Probe fokussierten Elektronenstrahls;
d) Verschieben der Probe in dem Neigungswinkel, um den Fokus des Elektronenstrahls zu mindestens einer zweiten Stelle auf der Fläche der Probe zu bewegen;
e) Detektieren des an mindestens der zweiten Stelle auf der Fläche der Probe fokussierten Elektronenstrahls; und
Wiederholen der Schritte b) bis e) bei einem oder mehreren unterschiedlichen Neigungswinkeln.

13. Elektronenmikroskopiesystem nach Anspruch 12, ferner
umfassend: einen Prozessor; und
Datenspeicher, in denen ausführbare Anweisungen gespeichert werden, die, wenn sie vom Prozessor ausgeführt werden, das Elektronenmikroskopiesystem konfigurieren, um Folgendes durchzuführen:
Erzeugen eines Computertomographie-Bildes der Probe unter Verwendung von Daten, die beim Detektieren des Elektronenstrahls erhalten werden.

14. Elektronenmikroskopiesystem nach Anspruch 13, wobei die Steuereinheit ferner konfiguriert ist zum:
f) Messen eines ersten Abstands zwischen dem ersten Merkmal und dem zweiten Merkmal senkrecht zum Elektronenstrahl, wobei die Probe in dem Neigungswinkel geneigt ist;
g) Messen eines zweiten Abstands zwischen dem ersten Merkmal und dem zweiten Merkmal senkrecht zum Elektronenstrahl, wobei die Probe in dem einen oder den mehreren unterschiedlichen Neigungswinkeln geneigt ist;
und
h) Berechnen einer Differenz im Abstand des ersten Merkmals und des zweiten Merkmals von der Fläche der Probe auf Grundlage einer Differenz zwischen dem ersten Abstand und dem zweiten Abstand und einer Differenz zwischen dem Neigungswinkel und dem einen oder den mehreren unterschiedlichen Neigungswinkeln.

15. Elektronenmikroskopiesystem nach Anspruch 13 oder Anspruch 14, wobei der Probenverschiebungstisch ferner eine Vielzahl von Elektromotoren umfasst.

## Revendications

1. Procédé permettant d'obtenir des données de tomographie électronique à partir d'un échantillon, ledit procédé comprenant les étapes suivantes :
a) focaliser un faisceau d'électrons sur un premier emplacement de la surface de l'échantillon ;
b) incliner la surface de l'échantillon selon un angle d'inclinaison par rapport au faisceau d'électrons, tout en maintenant la surface de l'échantillon dans le plan focal du faisceau d'électrons ;
c) détecter le faisceau d'électrons focalisé en un premier emplacement à la surface de l'échantillon ;
d) déplacer l'échantillon selon l'angle d'inclinaison afin de déplacer le plan focal du faisceau d'électrons vers au moins un deuxième emplacement de la surface de l'échantillon ;
e) détecter le faisceau d'électrons focalisé sur au moins le deuxième emplacement à la surface de l'échantillon ; et
répéter les étapes b) à e) selon un ou plusieurs angles d'inclinaison différents.

2. Procédé selon la revendication 1, comprenant en outre l'étape consistant à générer une image par tomographie assistée par ordinateur de l'échantillon à partir des données obtenues lors de la détection du faisceau d'électrons.

3. Procédé selon l'une quelconque des revendications précédentes, dans lequel l'angle d'inclinaison est un angle non normal par rapport au faisceau d'électrons.

4. Procédé selon l'une quelconque des revendications précédentes, dans lequel une première caractéristique de l'échantillon est située à un premier emplacement et une deuxième caractéristique de l'échantillon est située à un deuxième emplacement.

5. Le procédé selon la revendication 4, comprenant en outre les étapes suivantes :
f) mesurer une première distance entre la première caractéristique et la deuxième caractéristique, perpendiculairement au faisceau d'électrons, l'échantillon étant incliné selon l'angle d'inclinaison ;
g) mesurer une deuxième distance entre la première caractéristique et la deuxième caractéristique, perpendiculairement au faisceau d'électrons, l'échantillon étant incliné selon un ou plusieurs angles d'inclinaison différents ;
et
h) calculer la différence de distance entre la première caractéristique et la deuxième caractéristique par rapport à la surface de l'échantillon, en se basant sur la différence entre la première distance et la deuxième distance, ainsi que sur la différence entre l'angle d'inclinaison et le ou les autres angles d'inclinaison.

6. Procédé selon la revendication 5, dans lequel l'angle d'inclinaison ou le ou les angles d'inclinaison différents sont perpendiculaires à un axe du faisceau d'électrons.

7. Procédé selon l'une quelconque des revendications précédentes, dans lequel l'échantillon est un échantillon lamellaire.

8. Procédé selon l'une quelconque des revendications précédentes, comprenant en outre les étapes suivantes :
après l'étape c) et avant l'étape d), régler le faisceau d'électrons de manière à le focaliser sur un troisième emplacement ; et détecter le faisceau d'électrons focalisé en un troisième emplacement à la surface de l'échantillon.

9. Procédé selon l'une quelconque des revendications précédentes, comprenant en outre les étapes suivantes :
après l'étape d) et avant ou après l'étape e), ajuster le faisceau d'électrons pour le focaliser sur un quatrième emplacement ; et
détecter le faisceau d'électrons focalisé au quatrième emplacement à la surface de l'échantillon.

10. Procédé selon l'une quelconque des revendications précédentes, dans lequel la différence angulaire entre l'angle d'inclinaison et le ou les autres angles d'inclinaison est comprise entre 0,1 et 10 degrés.

11. Procédé selon l'une quelconque des revendications précédentes, dans lequel l'échantillon est déplacé selon l'angle d'inclinaison afin de déplacer la position du faisceau d'électrons sur l'échantillon d'une distance comprise entre 1 et 3 µm.

12. Système de microscopie électronique comprenant :
une source de faisceau d'électrons ;
un système optique de focalisation du faisceau d'électrons conçu pour focaliser ce faisceau sur la surface d'un échantillon ;
un plateau de transfert de l'échantillon conçu pour déplacer l'échantillon dans un plan perpendiculaire au faisceau d'électrons et pour faire varier un angle d'inclinaison de la surface de l'échantillon par rapport à un axe du faisceau d'électrons ; et **caractérisé par**
une unité de commande en liaison avec le plateau de transfert de l'échantillon et le système optique de focalisation du faisceau d'électrons, et conçue pour exécuter les étapes suivantes :
a) focaliser le faisceau d'électrons en un premier emplacement sur la surface de l'échantillon ;
b) incliner la surface de l'échantillon selon un certain angle par rapport au faisceau d'électrons tout en maintenant cette surface dans le plan focal du faisceau d'électrons ;
c) détecter le faisceau d'électrons focalisé en un premier emplacement à la surface de l'échantillon ;
d) déplacer l'échantillon selon l'angle d'inclinaison de manière à déplacer le plan focal du faisceau d'électrons vers au moins un deuxième emplacement de la surface de l'échantillon ;
e) détecter le faisceau d'électrons focalisé sur au moins le deuxième emplacement à la surface de l'échantillon ; et
répéter les étapes b) à e) en adoptant un ou plusieurs angles d'inclinaison différents.

13. Le système de microscopie électronique selon la revendication 12,
comprenant en outre : un processeur ; et
mémoire contenant des instructions exécutables qui, lorsqu'elles sont exécutées par le processeur, permettent de
configurer le système de microscopie électronique pour effectuer :
générer une image par tomographie assistée par ordinateur de l'échantillon à partir des données obtenues lors de la détection du faisceau d'électrons.

14. Système de microscopie électronique selon la revendication 13, dans lequel l'unité de commande est en outre
configurée pour :
f) mesurer une première distance entre la première caractéristique et la deuxième caractéristique, perpendiculairement au faisceau d'électrons, l'échantillon étant incliné selon l'angle d'inclinaison ;
g) mesurer une deuxième distance entre la première caractéristique et la deuxième caractéristique, perpendiculairement au faisceau d'électrons, l'échantillon étant incliné selon un ou plusieurs angles d'inclinaison différents ; et
h) calculer la différence de distance entre la première caractéristique et la deuxième caractéristique par rapport à la surface de l'échantillon, en se basant sur la différence entre la première distance et la deuxième distance, ainsi que sur la différence entre l'angle d'inclinaison et le ou les autres angles d'inclinaison.

15. Système de microscopie électronique selon la revendication 13 ou la revendication 14, dans lequel le plateau de transfert de l'échantillon comprend en outre plusieurs moteurs électriques.
